# EUROPEAN PATENT APPLICATION

(11) **EP 4 437 946 A1**
(43) Date of publication of application: **02.10.2024**
(21) Application number: 23165362.7
(22) Date of filing: 30.03.2023
(51) Int. Cl.: A61B 5/0507, A61B 5/0531, A61B 5/0536, A61B 5/00

(54) **DETECTING OBSTRUCTION OF DUCTS, NODES OR GLANDS IN SUBSURFACE TISSUE**

(71) Applicant: Koninklijke Philips N.V., 5656 AG Eindhoven (NL)
(72) Inventor: JOHNSON, Mark Thomas, Eindhoven (NL); PERRONE, Antonio Luigi, Eindhoven (NL); MENA BENITO, Maria Estrella, Eindhoven (NL); VAREKAMP, Christiaan, Eindhoven (NL)
(74) Representative: Philips Intellectual Property & Standards

(57) **Abstract**

Proposed are concepts pertaining to aiding detection of (partial or full) obstruction (i.e. at least partial blockage) of ducts, nodes or glands in tissue of a subject. In particular, embodiment of the invention proposed the use of microwave imaging. In this way, early detection and/or assessment of an obstruction of a duct, node or gland may be facilitated by proposed embodiments.

## Description

### FIELD OF THE INVENTION

The present invention relates to the field of health care, and in particular to the field of detecting obstruction of ducts, nodes or glands in subsurface tissue.

### BACKGROUND OF THE INVENTION

Mastitis and acne are typically due to the presence of blocked ducts in tissue. Blocked (or obstructed) milk ducts in breast tissue can cause mastitis. Likewise, (partially or fully) blocked sebum ducts or sebaceous glands in skin tissue may cause acne.

Early detection and diagnosis of blocked or obstructed milk ducts in breast tissue can therefore help to prevent mastitis (as the information can be used to alert the subject about the onset of milk statis, for example). Similarly, early detection of the onset of acne may help a subject (i.e. patient) to seek medical assistance before acne is visible (and consequently help to avoiding deeper skin inflammation).

Nonetheless, early detection and assessment procedures are not routinely performed, leading, usually, to a late diagnosis and late treatment recommendation.

Since blocked ducts, initially, don't show any external sign, there is a need for an approach to detect and monitor obstruction of ducts, nodes or glands in subsurface tissue at an early stage.

### SUMMARY OF THE INVENTION

The invention is defined by the claims.

According to examples in accordance with an aspect of the invention, there is provided a system for detecting obstruction of ducts, nodes or glands in subsurface tissue of a subject, the system comprising:
a microwave image acquisition unit configured to acquire at least one microwave image of a region of subsurface tissue of the subject; and
an analysis unit configured to analyze variations in the at least one microwave image to detect a clogging feature in the subsurface tissue of the subject, the clogging feature being indicative of an obstruction in a duct, node or gland in the subsurface tissue.

Proposed concepts thus aim to provide schemes, solutions, concepts, designs, methods and systems pertaining to aiding detection of (partial or full) obstruction (i.e. at least partial blockage) of ducts, nodes or glands in tissue of a subject through the use of microwave imaging. In this way, early detection and/or assessment of an obstruction of a duct, node or gland may be facilitated by proposed embodiments.

Embodiments may detect a clogging feature in subsurface tissue of a subject, and the clogging feature may be indicative of an obstruction (part or full) in a duct, node or gland in the subsurface tissue. By identifying part or full obstructions, solutions may be identified prior to occurrence of problems/issues, potentially simplifying treatment and/or facilitating preventative action(s).

The proposed concept(s) may also enhance reliability of the medical device development phase by providing information about faults and associated solutions that can be leverage to help avoid or prevent occurrence of the faults in the device usage phase.

In particular, embodiments of the invention propose using microwave images of a region of subsurface tissue of the subject. That is, embodiments may employ microwave imaging to obtain information that can be used to identify one or more clogging features in the subsurface tissue of the subject, the clogging feature being indicative of an obstruction in a duct, node or gland in the subsurface tissue.

Accordingly, the proposed concept(s) may benefit from the use of active microwave imaging to detect the onset of blocked ducts and/or glands.

In particular, it has been realized that, in terms of microwave frequencies, obstructed or blocked ducts and non-blocked ducts absorb microwaves differently. That is, it is proposed that reflected microwave's electrical properties, namely the permittivity and conductivity of the studied clogged tissues, differ from those of healthy tissues within the microwave band. Leveraging this, embodiments propose the use of a microwave imaging system (e.g. a miniaturized microwave camera) together with the associated computational system for microwave signal processing.

Embodiments may therefore enable early detection and diagnosis of obstructed or blocked ducts, nodes or glands. This may in true help a user to prevent the development of the obstruction/blockage, thereby avoiding associated problems (e.g. mastitis, abscess, acne, etc.). Embodiments may also help to increase awareness of the development of obstructions or blockages.

In some embodiments, the analysis unit may comprise: an image processing unit configured to process the at least one microwave image with a detection algorithm, the detection algorithm being adapted to identify variations in the at least one microwave image that meets a predetermined requirement; and a detection unit configured to detect the presence of a clogging feature based the identified variations. In this way, embodiments may leverage the realization that, in terms of microwave frequencies, blocked and non-blocked ducts/nodes/glands absorb microwaves differently. Through relatively simple analysis of variations in microwave image data of a subject's tissue, presence of a clogging feature in the tissue may be detected.

For instance, the at least one microwave image may comprise a plurality of temporally separated microwave images of the same subsurface tissue of the subject, and the predetermined requirement may be that a difference between a same portion of the temporally separated images exceeds a predetermined threshold value. The difference may, for example, comprise a difference in frequency response to microwave radiation at a target wavelength range of interest.

In another example, the at least one microwave image may comprise a plurality of temporally separated microwave images of the same subsurface tissue of the subject, and wherein the predetermined requirement may be that a least a part of the frequency spectrum for a same feature in the temporally separated images has shifted from a first wavelength to a second, different wavelength. For instance, the second wavelength may be substantially half or double the first wavelength.

In an embodiment, the analysis unit may comprise a machine learning algorithm configured to receive the at least one microwave image, the machine learning algorithm being trained to output an indication of a clogging feature in the subsurface tissue of the subject. The machine learning algorithm may be trained using a training algorithm configured to receive an array of training inputs and respective known outputs, wherein a training input comprises a microwave image of a training region of subsurface tissue of a subject, and wherein a respective known output comprises an indication of a known clogging feature in the training region of the subsurface tissue. In this way, the machine learning algorithm may be trained to output an indication of a clogging feature in subsurface tissue when provided with a microwave image of the subsurface tissue. Previous information regarding the presence of obstructions or blockages in subsurface tissue may thus be learnt and leveraged to make future predictions of the presence of obstructions/blockages in a subject's body tissue.

In some embodiments, the microwave image acquisition unit may comprise a microwave imaging system configured to generate at least one microwave image of a region of subsurface tissue of the subject. That is, some embodiments may be adapted to generate the one or more microwave images. In this way, real-time assessment and detection may be facilitated.

Conversely, other embodiments may be adapted to use microwave images generated by an external microwave imaging unit (e.g. retrieve or otherwise obtain the one or more microwave images from a database or microwave imaging device). In this way, embodiments may be employed in conjunction with historical (i.e. previously obtained) microwave images.

In an exemplary embodiment, the at least one microwave image may comprise: a microwave image comprising a plurality of pixels corresponding to a respectively plurality of sub-regions of the region of subsurface tissue, wherein each pixel comprises a pixel value representing a frequency response of the respective sub-region to microwave radiation.

In an embodiment, the analysis unit may be configured to process the at least one microwave interferometry algorithm to determine coherence values for the region of subsurface tissue of the subject. The analysis unit may then detect a clogging feature in the subsurface tissue of the subject based on the determined coherence values. In this way, known microwave interferometry techniques may be leveraged.

In another embodiment, the analysis unit may be configured to process the at least one microwave polarimetry algorithm to determine polarization values for the region of subsurface tissue of the subject. The analysis unit may then detect a clogging feature in the subsurface tissue of the subject based on the determined polarization values. Known microwave polarimetry techniques may therefore be employed by embodiments for the purposes of identifying clogging features in subsurface tissue.

The region of subsurface tissue may, for example, comprise breast tissue of the subject. The clogging feature may then be indicative of an obstruction in a milk duct of the breast tissue.

Alternatively, the region of subsurface tissue may comprise subsurface skin tissue of the subject. The clogging feature may then be indicative of an obstruction in a sebaceous gland of the subsurface skin tissue.

Embodiments may be employed in existing or conventional health care or consumer equipment, thus providing new and/or extended functionalities. For instance, according to an aspect of the invention, there may be provided a personal care device comprising a system according to a proposed embodiment. The personal care device may, for example, comprise a breast pump, massage device or shaver.

Also, the system may be remotely located from a user device for detecting obstruction of ducts, nodes or glands in subsurface tissue of a subject. In this way, a user (such as a patient, consumer or medical practitioner) may have an appropriately arranged system that can receive information about detected clogging features in the subject's subsurface tissue at a location remotely located from the system. Embodiments may therefore enable a user to identify and/or assess obstructions of ducts, nodes or glands in subsurface tissue using a local system (which may, for example, comprise a portable display device, such as a laptop, tablet computer, mobile phone, PDA, etc.). By way of example, embodiments may provide an application for a mobile computing device, and the application may be executed and/or controlled by a user of the mobile computing device.

The system may further include: a server device comprising the system detecting obstruction of ducts, nodes or glands in subsurface tissue of a subject; and a client device comprising a user-interface. Dedicated data processing means may therefore be employed for the purpose of detecting clogging features in subsurface tissue that are indicative of obstructions in duct, nodes or glands, thus reducing processing requirements or capabilities of other components or devices of the system.

The system may further include a client device, wherein the client device comprises the analysis unit, and a display unit. In other words, a user (such as a service or development engineer) may have an appropriately arranged client device (such as a laptop, tablet computer, mobile phone, PDA, etc.) which processes received data in order to detect clogging features in subsurface tissue and generate a display control signal. Purely by way of example, embodiments may therefore provide a system that enables identification of an obstruction in a duct, node or gland, wherein real-time communication between a user (e.g. patient or medical practitioner) and a microwave imaging device is provided and can have its functionality extended or modified according to proposed concepts, for example.

It will be understood that processing capabilities may therefore be distributed throughout the system in different ways according to predetermined constraints and/or availability of processing resources.

One or more concepts for detecting obstruction of ducts, nodes or glands in subsurface tissue of a subject are thus provided, and these may enable the pre-emptive identification of (part or full) blockages earlier than conventional approaches.

According to another aspect of the invention, there is provided a method for detecting obstruction of ducts, nodes or glands in subsurface tissue of a subject, the method comprising: acquiring at least one microwave image of a region of subsurface tissue of the subject; and analyzing variations in the at least one microwave image to detect a clogging feature in the subsurface tissue of the subject, the clogging feature being indicative of an obstruction in a duct, node or gland in the subsurface tissue.

According to another aspect, there is provided a computer program product, wherein the computer program product comprises a computer-readable storage medium having computer-readable program code embodied therewith, the computer-readable program code configured to perform all of the steps of a proposed embodiment.

Thus, there may also be provided a computer system comprising: a computer program product according to proposed embodiment; and one or more processors adapted to perform a method according to a proposed concept by execution of the computer-readable program code of said computer program product.

One or more concepts are provided for predicting/deriving un-foreseen faults of a medical device problems, and this may support improved medical diagnostics or treatment. By way of example, proposed embodiments may address the issue of having to wait for outwardly visible symptoms to occur before an obstruction or blockage of a duct, node or gland can be detected. By detecting obstruction or blockage of a duct, node or gland earlier, improved treatment and/or preventative measures may be undertaken.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 depicts a simplified block diagram of a system for detecting obstruction of ducts, nodes or glands in subsurface tissue of a subject according to an embodiment;
Fig. 2 is a simplified diagram illustrating ducts in subsurface tissue, wherein the wavelengths of absorbed and reflected microwaves are depicted;
Fig. 3 depicts a simplified block diagram of a system for detecting obstruction of ducts, nodes or glands in subsurface tissue of a subject according to another embodiment;
Fig. 4 depicts is a flow diagram of a method for detecting obstruction of ducts, nodes or glands in surface tissue of a subject according to an embodiment; and
Fig. 5 is a simplified block diagram of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

It should be understood that the Figures are merely schematic and are not drawn to scale. It should also be understood that the same reference numerals are used throughout the Figures to indicate the same or similar parts.

The invention proposes concepts for detection (partial or full) obstruction of ducts, nodes or glands in body tissue of a subject. In particular, embodiments may provide a method and/or system which helps to detect a clogging feature in subsurface tissue of a subject and this may facilitate the early identification of an obstruction of a duct, node or gland of a subject. Proposed embodiments may therefore simplify medical treatment and/or facilitate preventative action(s).

Proposed concepts may be based on a realization that, in terms of microwave frequencies, obstructed or blocked ducts and non-blocked ducts absorb microwaves differently. Microwave images of a region of subsurface tissue of the subject may thus be analyzed to identify one or more clogging features in the subsurface tissue of the subject, the clogging feature being indicative of an obstruction in a duct, node or gland in the subsurface tissue.

Accordingly, embodiments may be used in relation to detection and diagnosis of obstructed or blocked ducts, nodes or glands of a subject.

Proposed embodiments may thus facilitate improved (e.g. quicker and/or easier) obstruction or blockage detection through the detection of clogging features based on variations in one or more microwave images of a subject's body tissue. Embodiments of the present invention are therefore directed toward enabling the improved searching of potential obstructions of ducts, nodes or glands in subsurface tissue.

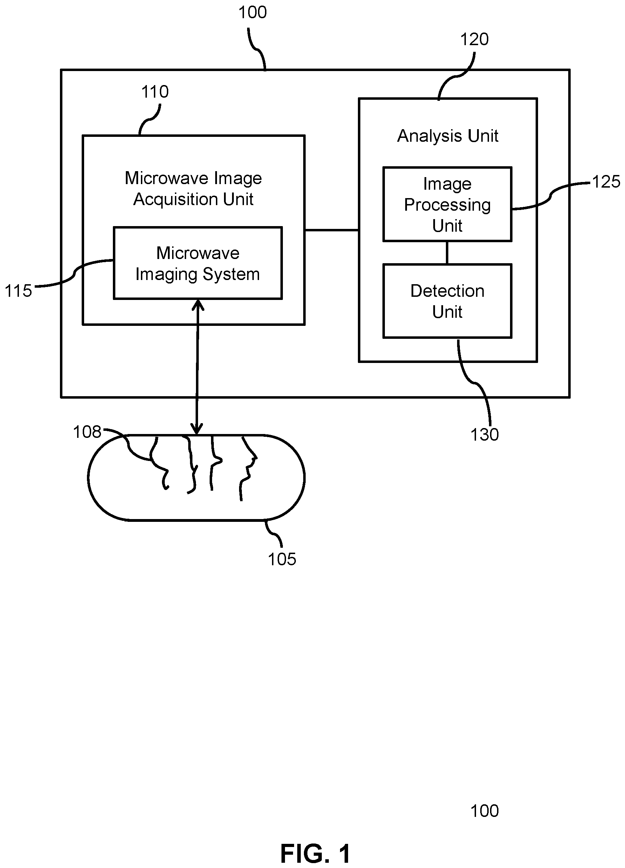

Fig. 1 illustrates an embodiment of a system 100 for detecting obstruction of ducts, nodes or glands in subsurface tissue 105 of a subject. Specifically, the region of subsurface tissue 105 comprises breast tissue of the subject, and the embodiment is configured to detect clogging features indicative of an obstruction in a milk duct 108 of the breast tissue 105.

The system 100 comprises a microwave image acquisition unit 110 that is configured to acquire at least one microwave image of a region of the breast tissue 105 of the subject. Here, a microwave image comprises a plurality of pixels corresponding to a respectively plurality of sub-regions of the region of breast tissue 105. Each pixel comprises a pixel value representing a frequency response of the respective sub-region to microwave radiation.

In this embodiment, the microwave image acquisition unit comprises a microwave imaging system 115 that is configured to generate at least one microwave image of a region of breast tissue 105 of the subject. The exemplary embodiment of Fig. 1 thus comprises an appropriately adapted microwave imaging system 115 to generate the required microwave images of the subject's breast tissue 105. Suitable microwave imaging systems are widely known and available, with some even being of size suitable for incorporation within a personal/portable health care device.

The system 100 also comprises an analysis unit 120 that is configured to analyze variations in the at least one microwave image to detect a clogging feature in the subsurface tissue of the subject, wherein the clogging feature is indicative of an obstruction in a duct, node or gland in the subsurface tissue.

In this embodiment, the analysis unit comprises an image processing unit 125 and a detection unit 130. The image processing unit 125 is configured to process the at least one microwave image with a detection algorithm. The detection algorithm is adapted to identify variations in the at least one microwave image that meets a predetermined requirement. The detection unit 130 is then configured to detect the presence of a clogging feature based the identified variations.

In more detail, the at least one microwave image in the embodiment of Fig. 1 comprises a plurality of temporally separated microwave images of the same breast tissue 105 of the subject. The predetermined requirement then requires that a difference between a same portion of the temporally separated images exceeds a predetermined threshold value. Specifically, the difference is a difference in frequency response to microwave radiation at a target wavelength range of interest. In this way, if a change in frequency response for the same portion of breast tissue over time exceeds a predefined amount (i.e. threshold value), the presence of a clogging feature in that portion of breast tissue may be inferred. Such a clogging feature may be indicative of an obstruction (partial of full) of a milk duct 108 in the breast tissue 105.

Variations to the above-described embodiment of Fig. 1 may include employing different approaches to obtaining the microwave images. For example, in an alternative embodiment, the microwave image acquisition unit may be adapted to receive or retrieve the at least one microwave image from an external/remote imaging system or microwave image database (via in input interface). That is, an alternative embodiment may acquire microwave images from a source external to the system.

By way of yet another example, in some embodiments the at least one microwave image comprises a plurality of temporally separated microwave images of the same subsurface tissue of the subject. The predetermined requirement then requires that a least a part of the frequency spectrum for a same feature in the temporally separated images has shifted from a first wavelength to a second, different wavelength. Preferably the second wavelength may be substantially half or double the first wavelength. Such an example may help to identify resonance of microwaves in closed ducts (e.g. based on the proposal that a blocked duct will absorb at half the wavelength of an open duct) or ducts which become unblocked (e.g. based on the proposal that an open duct will absorb at double the wavelength of a closed duct).

As detailed above, it is proposed to detect clogging features in subsurface tissue of a subject by analyzing variations in one or more microwave images of the tissue. The above example details application of the proposed concept(s) to breast tissue of a subject. However, in other embodiments the region of subsurface tissue may comprise subsurface skin tissue, and a detected clogging feature may then be indicative of an obstruction in a sebaceous gland of the subsurface skin tissue for example. That is, embodiments are not limited to the detection of blocked/obstructed milk ducts, and may instead by used to detect obstructions of other subsurface tissue features such as glands, nodes and the like.

Proposed embodiments employ microwave images, created using electromagnetic waves in the microwave regime (i.e., ~300 MHz-300 GHz).

In general, a microwave imaging system is typically made up of hardware and software components. The hardware collects data from the sample under test (e.g. body tissue). A transmitting antenna sends electromagnetic (EM) waves towards the sample under test (e.g., human body tissue for medical imaging). If the sample is made of only homogeneous material and is of infinite size, theoretically no EM wave will be reflected. Introduction of any anomaly which has different properties (i.e., electro-magnetic) in comparison with the surrounding homogeneous medium will reflect a portion of the EM wave.

It is proposed that the reflected EM wave's electrical properties, namely the permittivity and conductivity of the studied clogged tissues, differ from those of healthy tissues within the microwave band. The bigger the difference between the properties of the anomaly and the surrounding medium is, the stronger the reflected wave will be. This reflection may be collected by the same antenna in a monostatic system, or a different receiver antenna in bistatic configurations.

It is known that the microwave impedance of air, fluid (milk/sebum/lymph etc.), tissue, and blockages will be different (see Table 1 below which details water content and complex permittivity of different components of the analyzed anatomies).

**Table 1.**

| COMPARTMENT | WATER CONTENT | BOUND WATER | COMPLEX PERMITTIVITY |
|---|---|---|---|
| EPIDERMIS AND DERMIS | - | - | 8.17-11.27j |
| ARRECTOR PILI MUSCLES | - | - | 12.9-15.8j |
| HAIR | - | - | 2.6-0.1j |
| WATER | - | - | 15.2-23.3j |
| LYMPH | 96.6% | 1.1% | 14.84-22.3j |
| SWEAT | 97.79% | 0.7% | 15.02-22.7j |
| SEBACEOUS GLANDS | 13.2% | 22.5% | 4.4-1.7j |
| HUMID HAIR | 0.1% | 32.1% | 3.34-0.1j |
| HUMAN BREAST MILK | 90% | - | 12.75-18.7j (estimated) |

For reference, as shown in Table 1, human breast milk (HBM) contains about 87%-88% water, and 124- g/L solid components as macronutrients, including about 7% (60-70 g/L) carbohydrates, 1% (8-10 g/L) protein, and 3.8% (35-40 g/L) fat. The HBM dielectric properties are distinctively different from the other surrounding tissues (e.g., average decaying time in a Cole-Cole model of about 162±10 ns). For example, whilst air has the lowest impedance, fluid will have a higher impedance and a blockage (which for milk ducts is a much more compacted plug of dried out Fat cells) an even higher impedance. These different permittivity properties of blocked ducts (in a tube) and non- blocked ones (fluid in non-blocked tub) may be probed by the microwaves.

Also, it is proposed that ducts and glands will act as microwave antennas. Microwaves will resonate in these structures in an analogous manner to acoustic waves in an organ pipe.
- An open duct will resonate when a quarter wavelength fits to the length of the duct i.e., when the microwave has a wavelength of four (4) times the duct length.
- Conversely, because of the higher impedance of an obstruction or blockage, an obstructed/blocked duct will appear to be closed to the microwave. A closed duct will resonate when a half wavelength fits to the length of the duct i.e., when the microwave has a wavelength of two (2) times the duct diameter. Consequently, a blocked duct will absorb at half the wavelength of an open duct.

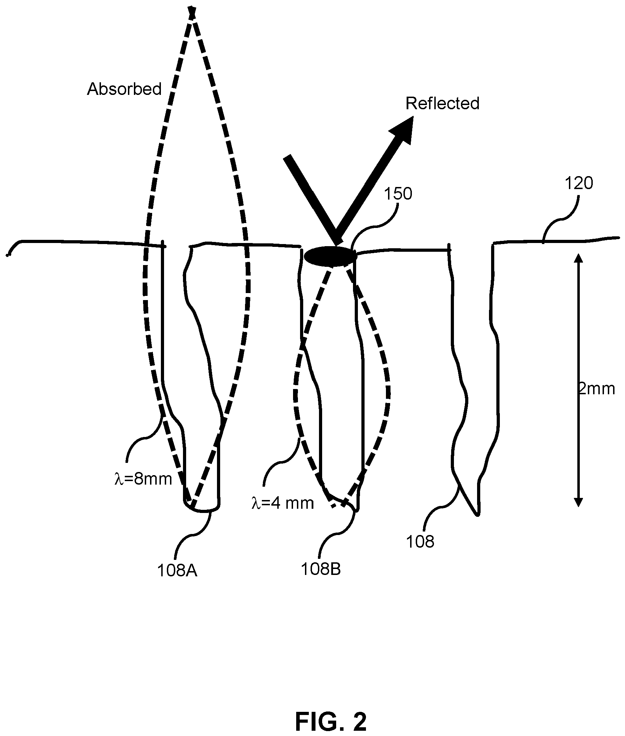

For example, Fig. 2 is a simplified diagram illustrating ducts 108 in subsurface tissue, wherein the wavelengths of absorbed and reflected microwaves are depicted using dashed lines. The illustrated ducts are 2 mm in length.

As depicted for the left-most duct 108A (i.e. an open duct) the duct 108A resonates when a quarter wavelength fits to the length of the duct 108A. That is, the microwave has a wavelength of four (4) times the duct length, specifically 8 mm in the example of Fig. 2. Conversely, as depicted for the middle duct 108B (i.e. an obstructed/blocked duct), because of the higher impedance of an obstruction or blockage, the obstructed/blocked duct 108B appears to be closed to the incident microwave. The blocked duct 108B resonates when a half wavelength fits to the length of the duct 108B. That is, when the microwave has a wavelength of two (2) times the duct length, specifically 4 mm in the example of Fig. 2.

It will therefore be appreciated that the obstructed/blocked duct 108B absorb microwaves at half the wavelength of the open duct 108A.

Proposed embodiments may therefore be configured to detect the presence of clogging features by analyzing the frequency spectrum for subsurface tissue feature across temporally separated microwave images. If a shift from a first wavelength to a second wavelength (that is half the first wavelength) is detected, the presence of an obstruction or blockage may be inferred. Similarly, a shift to a wavelength that is double the first wavelength is indicative that a blockage may have been cleared.

The above-described microwave imaging device is a typical configuration of a radar-system, usually comprising a single antenna. However, there is another, alternative, main category for the antennas' configuration, the tomographic one, that may be employed by proposed embodiments.

It is also noted that microwave imaging systems may be incorporated into personal care and healthcare devices (e.g. breast pumps, massage devices, shavers or epilators). Embodiments may thus support imaging-assisted care device that may enable remote image-based diagnostics, evaluation, detection, classification, management, therapy planning or treatment monitoring.

By way of further description of the proposed concept(s), an embodiment may be summarized as comprising the following elements:
(I) An active microwave imaging system / Microwave Imaging unit: The dimensions of ducts are typically around several mm in length and around 1-3mm in diameter. These are typical microwave wavelengths. Examples of such wideband, multispectral microwave cameras are for example:
   - (a) The MIT microwave camera: when it takes each measurement, the microwave emitter sweeps through a frequency range of 7.835 GHz to 12.817 GHz over 10 ms. Different materials respond to the microwaves differently at lower and higher frequencies, and the camera can separate out these spectra. This provides an image with multiple frequency response "colors," and the patterns of colors provides information about the materials.
   - (b) A system proposed by Pagliari et al. operates over a relatively small bandwidth of 200 MHz between 1.4 and 1.6 GHz. The relatively low cost for this system was maintained due to the availability of low-cost off-the-shelf components in the working frequency range of the system. The system has demonstrated capability to localize precisely a 12mm metal target embedded in a breast phantom.
(II) A Computational unit: microwave images are gathered and then analyzed using a dedicated imaging algorithm to detect the possible presence of a clogging feature indicative of an obstruction or blockage. By way of example, the algorithm may be adapted to identify the development of duct related clogging features developing at half the wavelength of that in previously microwave images of the same tissue.

By way of further description, various alternative approaches to employing the proposed concept(s) will now be described in the following sections.

### A. Microwave Tomography

In microwave imaging, the tomography approach is typically performed iteratively and can be represented by a nonlinear inverse problem that requires significant computational resources for producing the dielectric properties of the tissue under investigation. Imaging of the tissues can be constructed from the recovered microwave data file through inverse scattering algorithms that estimate the constitutive parameters of the tissues by analyzing the absorbed and reflected microwave signals (as described above).

In this embodiment, it is proposed to reconstruct the detailed 3D shape of the body tissue under observation using a reconstruction algorithm (e.g., local - conjugate gradient least squares (CGLS) algorithm, least squares QR (LSQR) algorithm, and Landweber algorithm - or global - genetic algorithm (GA) and particle-swarm optimization (PSO) algorithm - gradient based approaches). This reconstruction includes the complex permittivity values as reconstructed from the tomograms received through a set of microwave transceivers arranged in a geometric shape (circular etc.).

### B. Microwave Interferometry

Microwave interferometry is known in the field of radar remote sensing. It is proposed that interferometry can also be used for detection of blocked or obstructed ducts.

For instance, using two antennas separated by a known baseline, one antenna can transmit a pulse while both antennas receive the backscatter from the skin tissue and subsurface tissue. Both the position and the reflectivity of objects in the tissue will determine the phase difference and coherence magnitude of the so-called complex coherence (correlation) of the two signals that are received by the two antennas.

In the case that backscatter results from a single isolated point, then the coherence magnitude equals one (1) and the phase difference depends on the position of the point. However, if many scatterers at different positions contribute to the received signal, then the coherence magnitude will be closer to zero (0) and the phase difference will correspond with a weighted average position of the scatterers.

It is proposed that duct blockages/obstructions are small and strong scatterers, thus resulting in a relatively higher coherence magnitude.

The imaging mode for spatially calculating the complex coherence may be achieved by moving two antennas, or alternatively by using a true imaging system (e.g., using a synthetic aperture). When using an imaging system, it may be possible to calculate the 3D position of the duct blockage by interpretation of the phase difference - known techniques from radar remote sensing can be used to do this. Notably, two-dimensional phase unwrapping techniques may be used to determine the (unwrapped) absolute phase that is needed for calculation of the 3D position.

### C. Microwave Polarimetry

Another known technique from radar remote sensing is polarimetry. Vertical or horizontally polarized waves are transmitted, and the horizontal and vertical components of the backscattered wave are measured.

It is known that at larger (centimeter) microwave lengths, the orientation of scatterers will cause received power differences for different combinations of linear transmit and receive polarizations.

It is proposed that duct blockages/obstructions will show a strong preference for a certain linear polarization (based on the expectation that the blockage/obstruction is not a linear structure). However, it may be that hair or other linear structures provide a similar backscatter as a blockage/obstruction. These linear structures may be distinguished from the blockage/obstruction by their particular polarization response.

Where it is difficult to predict the exact polarimetric scattering properties of sub-surface skin features, a practical approach may be to produce a dataset where another imaging modality is used as reference, or a visual interpretation is done by an expert who can interpret/annotate features based on false color polarimetric images.

### D. Polarimetric Interferometry

Polarimetric interferometry is a known remote sensing technique that uses two antennas in combination with polarimetric operation. It is proposed that it can also be employed for object detection in subsurface tissue.

If a hand-held device with two mounted antennas is used, the device orientation may be measured separately, and the polarization direction will change as a function of time. When combined with the device orientation measurements, the signals may be analyzed to obtain detailed polarimetric information without the need for a full imaging radar system (the scanning hand then essentially produces the imaging mode).

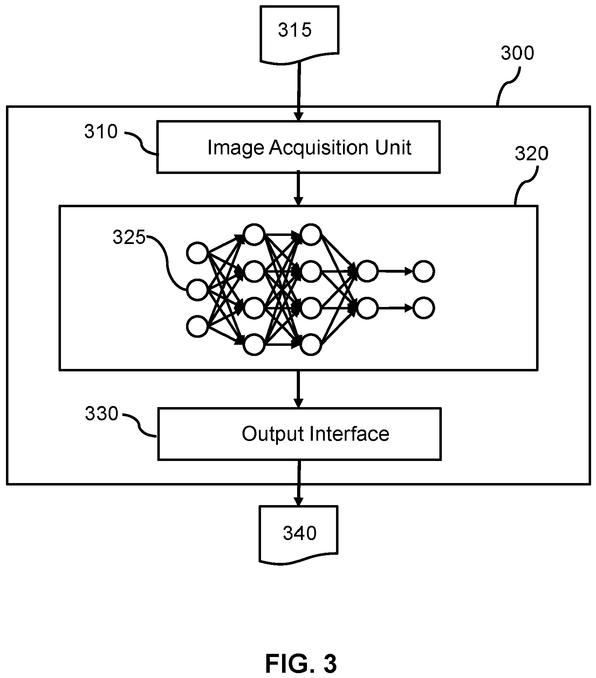

By way of summary of another proposed implementation, Fig. 3 depicts a simplified block diagram of a system 300 for detecting obstruction of ducts, nodes or glands in subsurface tissue of a subject.

The system 300 comprises a microwave image acquisition unit 310 that is configured to receive a microwave image of a region of subsurface tissue of the subject.
The system 300 also comprises an analysis unit 320 that is configured to analyze variations in the microwave image to detect a clogging feature in the subsurface tissue of the subject.

In this embodiment, the analysis unit comprises a machine learning algorithm 325 configured to receive the at microwave image, the machine learning algorithm being trained to output an indication of a clogging feature in the subsurface tissue of the subject.

Here, the machine learning algorithm 325 is a neural network (NN)-based machine learning algorithm. The machine learning algorithm is trained to predict a clogging feature in the subsurface tissue of the subject. The structure of an artificial NN (or, simply, neural network (NN)) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

There are several types of neural network, such as convolutional neural networks (CNNs) and recurrent neural networks (RNNs). However, the exemplary embodiment of Fig. 3 employs a CNN, because CNNs have been shown to be particularly advantages for image processing.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, weightings of the mathematical operation of each neuron may be modified until the error converges. Known methods of modifying a neural network include gradient descent, backpropagation algorithms and so on.

The training input data entries for the machine learning algorithm used correspond to example microwave images. The training output data entries correspond to indications of a known clogging features in the example microwave images. That is, the machine learning algorithm is trained using a training algorithm configured to receive an array of training inputs and respective known outputs, wherein a training input comprises: a microwave image of a training region of subsurface tissue of a subject; and wherein a respective known output comprises an indication of a known clogging feature in the training region of the subsurface tissue. In this way, the machine learning algorithm is trained to output an indication of a clogging feature in subsurface tissue when provided with a microwave image of the subsurface tissue.

By way of example, information from longitudinal data may be used to determine the probability of development a blocked duct (e.g. based on data related to how fast variations in microwave images happen). Because the scattering process of microwaves in (random) media depends on the sizes of objects relative to the wavelength and also on dielectric properties, reference datasets may be established for normal tissue and for tissue where abnormalities have occurred. 1D or 2D microwave signals may be hand-annotated, such that a per samples or per pixels classifier can be trained and later applied to identify blocked ducts.

The machine learning algorithm 325 is trained to output an indication of detected clogging features in the subsurface tissue of the subject.

The system 600 also comprises an output interface 330 that is configured to output the indication 340 of detected clogging features in the subsurface tissue of the subject.

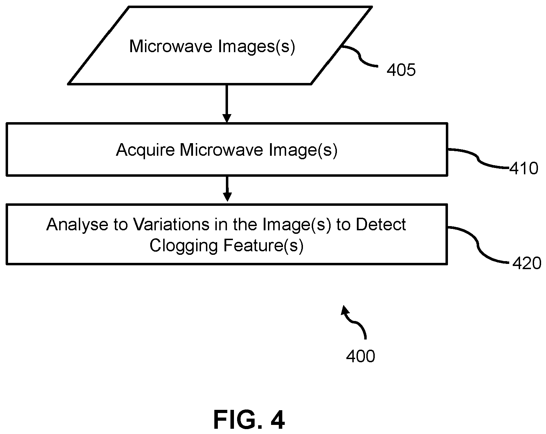

Fig. 4 illustrates a simplified flow diagram of a method for detecting obstruction of ducts, nodes or glands in subsurface tissue of a subject according to an embodiment.

The method begins with step 410 of acquiring at least one microwave image 405 of a region of subsurface tissue of the subject. Then, in step 420, variations in the at least one microwave image are analysed to detect a clogging feature in the subsurface tissue of the subject, the clogging feature being indicative of an obstruction in a duct, node or gland in the subsurface tissue.

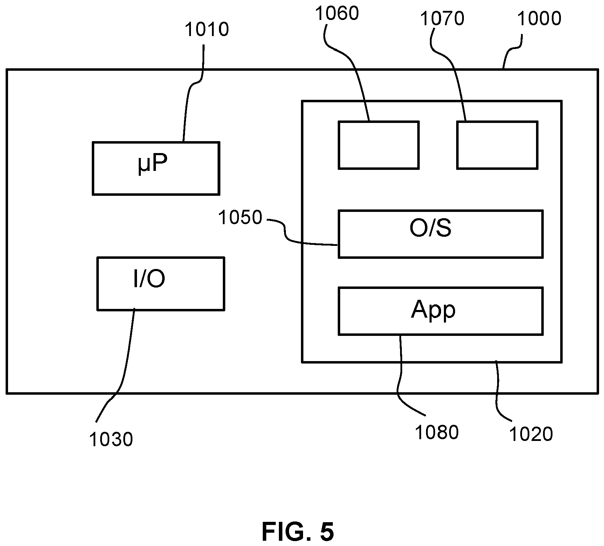

Fig. 5 illustrates an example of a computer 1000 within which one or more parts of an embodiment may be employed. Various operations discussed above may utilize the capabilities of the computer 1000. For example, one or more parts of a system for detecting obstruction of ducts, nodes or glands in subsurface tissue of a subject may be incorporated in any element, module, application, and/or component discussed herein. In this regard, it is to be understood that system functional blocks can run on a single computer or may be distributed over several computers and locations (e.g. connected via internet).

The computer 1000 includes, but is not limited to, PCs, workstations, laptops, PDAs, palm devices, servers, storages, and the like. Generally, in terms of hardware architecture, the computer 1000 may include one or more processors 1010, memory 1020 and one or more I/O devices 1030 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 1010 is a hardware device for executing software that can be stored in the memory 1020. The processor 1010 can be virtually any custom made or commercially available processor, a central processing unit (CPU), a digital signal processor (DSP), or an auxiliary processor among several processors associated with the computer 1000, and the processor 1010 may be a semiconductor-based microprocessor (in the form of a microchip) or a microprocessor.

The memory 1020 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 1020 may incorporate electronic, magnetic, optical, and/or other types of storage media. Note that the memory 1020 can have a distributed architecture, where various components are situated remote from one another, but can be accessed by the processor 1010.

The software in the memory 1020 may include one or more separate programs, each of which comprises an ordered listing of executable instructions for implementing logical functions. The software in the memory 1020 includes a suitable operating system (O/S) 1050, compiler 1060, source code 1070, and one or more applications 1080 in accordance with exemplary embodiments. As illustrated, the application 1080 comprises numerous functional components for implementing the features and operations of the exemplary embodiments. The application 1080 of the computer 1000 may represent various applications, computational units, logic, functional units, processes, operations, virtual entities, and/or modules in accordance with exemplary embodiments, but the application 1080 is not meant to be a limitation.

The operating system 1050 controls the execution of other computer programs, and provides scheduling, input-output control, file and data management, memory management, and communication control and related services. It is contemplated by the inventors that the application 1080 for implementing exemplary embodiments may be applicable on all commercially available operating systems.

Application 1080 may be a source program, executable program (object code), script, or any other entity comprising a set of instructions to be performed. When a source program, then the program is usually translated via a compiler (such as the compiler 1060), assembler, interpreter, or the like, which may or may not be included within the memory 1020, so as to operate properly in connection with the O/S 1050. Furthermore, the application 1080 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 1030 may include input devices such as, for example but not limited to, a mouse, keyboard, scanner, microphone, camera, etc. Furthermore, the I/O devices 1030 may also include output devices, for example but not limited to a printer, display, etc. Finally, the I/O devices 1030 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 1030 also include components for communicating over various networks, such as the Internet or intranet.

If the computer 1000 is a PC, workstation, intelligent device or the like, the software in the memory 1020 may further include a basic input output system (BIOS) (omitted for simplicity). The BIOS is a set of essential software routines that initialize and test hardware at start-up, start the O/S 1050, and support the transfer of data among the hardware devices. The BIOS is stored in some type of read-only-memory, such as ROM, PROM, EPROM, EEPROM or the like, so that the BIOS can be executed when the computer 800 is activated.

When the computer 1000 is in operation, the processor 1010 is configured to execute software stored within the memory 1020, to communicate data to and from the memory 1020, and to generally control operations of the computer 1000 pursuant to the software. The application 1080 and the O/S 1050 are read, in whole or in part, by the processor 1010, perhaps buffered within the processor 1010, and then executed.

When the application 1080 is implemented in software it should be noted that the application 1080 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 1080 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Fig. 4 and the systems of Figs. 1 and 3 may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Figs. 1 and 3 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A system for detecting obstruction of ducts, nodes or glands in subsurface tissue of a subject, the system comprising:
a microwave image acquisition unit configured to acquire at least one microwave image of a region of subsurface tissue of the subject; and
an analysis unit configured to analyze variations in the at least one microwave image to detect a clogging feature in the subsurface tissue of the subject, the clogging feature being indicative of an obstruction in a duct, node or gland in the subsurface tissue.

2. The system of claim 1, wherein the analysis unit comprises:
an image processing unit configured to process the at least one microwave image with a detection algorithm, the detection algorithm being adapted to identify variations in the at least one microwave image that meets a predetermined requirement; and
a detection unit configured to detect the presence of a clogging feature based the identified variations.

3. The system of claim 2, wherein the at least one microwave image comprises a plurality of temporally separated microwave images of the same subsurface tissue of the subject, and wherein the predetermined requirement is that a difference between a same portion of the temporally separated images exceeds a predetermined threshold value.

4. The system of claim 3, wherein the difference comprises a difference in frequency response to microwave radiation at a target wavelength range of interest.

5. The system of claim 2, wherein the at least one microwave image comprises a plurality of temporally separated microwave images of the same subsurface tissue of the subject, and wherein the predetermined requirement is that a least a part of the frequency spectrum for a same feature in the temporally separated images has shifted from a first wavelength to a second, different wavelength, and preferably wherein the second wavelength is substantially half or double the first wavelength.

6. The system of any of claims 1 to 5, wherein the analysis unit comprises a machine learning algorithm configured to receive the at least one microwave image, the machine learning algorithm being trained to output an indication of a clogging feature in the subsurface tissue of the subject,
and optionally wherein: the machine learning algorithm is trained using a training algorithm configured to receive an array of training inputs and respective known outputs, a training input comprising a microwave image of a training region of subsurface tissue of a subject, and a respective known output comprising an indication of a known clogging feature in the training region of the subsurface tissue.

7. The system of any of claims 1 to 6, wherein the microwave image acquisition unit comprises:
a microwave imaging system configured to generate at least one microwave image of a region of subsurface tissue of the subject.

8. The system of any of claims 1 to 7, wherein the at least one microwave image comprises: a microwave image comprising a plurality of pixels corresponding to a respective plurality of sub-regions of the region of subsurface tissue, wherein each pixel comprises a pixel value representing a frequency response of the respective sub-region to microwave radiation.

9. The system of any of claims 1 to 8, wherein the analysis unit is configured to process the at least one microwave interferometry algorithm to determine coherence values for the region of subsurface tissue of the subject, and to detect a clogging feature in the subsurface tissue of the subject based on the determined coherence values.

10. The system of any of claims 1 to 8, wherein the analysis unit is configured to process the at least one microwave polarimetry algorithm to determine polarization values for the region of subsurface tissue of the subject, and to detect a clogging feature in the subsurface tissue of the subject based on the determined polarization values.

11. The system of any of claims 1 to 10, wherein the region of subsurface tissue comprises breast tissue of the subject, and wherein the clogging feature is indicative of an obstruction in a milk duct of the breast tissue.

12. The system of any of claims 1 to 10, wherein the region of subsurface tissue comprises subsurface skin tissue of the subject, and wherein the clogging feature is indicative of an obstruction in a sebaceous gland of the subsurface skin tissue.

13. A personal care device comprising a system according to any of claims 1 to 12, and preferably wherein the personal care device comprises a breast pump, massage device or shaver.

14. A method for detecting obstruction of ducts, nodes or glands in subsurface tissue of a subject, the method comprising:
acquiring at least one microwave image of a region of subsurface tissue of the subject; and
analysing variations in the at least one microwave image to detect a clogging feature in the subsurface tissue of the subject, the clogging feature being indicative of an obstruction in a duct, node or gland in the subsurface tissue.

15. A computer program comprising code means for implementing the method of claim 14 when said program is run on a processing system.
